# EUROPEAN PATENT SPECIFICATION

(11) **EP 4 074 264 B1**
(45) Date of publication and mention of the grant of the patent: **07.05.2025**
(21) Application number: 20898049.0
(22) Date of filing: 07.12.2020
(51) Int. Cl.: A61B 10/02, A61B 10/04

(54) **BIOPSY NEEDLE AND TISSUE COLLECTION DEVICE**
BIOPSIENADEL UND GEWEBEENTNAHMEVORRICHTUNG
AIGUILLE DE BIOPSIE ET DISPOSITIF DE COLLECTE DE TISSU

(30) Priority: 13.12.2019 JP 2019225567
(43) Date of publication of application: 19.10.2022
(73) Proprietor: FUJIFILM Corporation, Tokyo 106-8620 (JP)
(72) Inventor: NISHIMURA, Tetsuya, Ashigarakami-gun, Kanagawa 258-8538 (JP)
(74) Representative: Dehns Germany Partnerschaft mbB
(86) International application number: PCT/JP2020/045362
(87) International publication number: WO 2021/117649

(56) References cited:
- WO-A1-2014/112518
- WO-A1-2019/157389
- JP-A- 2013 523 333
- US-A1- 2018 228 476

## Description

### BACKGROUND OF THE INVENTION

### 1. Field of the Invention

The present invention relates to a biopsy needle and a tissue collecting device, and more particularly to a biopsy needle and a tissue collecting device that are used to collect biological tissue.

### 2. Description of the Related Art

In recent years, in order to make a diagnosis, a needle tube has been introduced into a body cavity through a treatment tool-insertion channel of an ultrasonic endoscope, the needle tube has been guided to a portion to be observed and been inserted into lesional tissue under the observation of an ultrasonic tomographic image obtained performed by the ultrasonic endoscope, and biological tissue has been collected to confirm pathology.

For example, a tissue collecting device disclosed in WO2014/112518A is known as a tissue collecting device used to make such a pathological confirmation diagnosis. The tissue collecting device disclosed in WO2014/112518A comprises a flexible sheath that is freely inserted into a treatment tool-insertion channel of an endoscope, a needle tube that is inserted into the sheath to freely advance and retreat and punctures biological tissue, an operation unit that is connected to the proximal end portion of the sheath and allows the needle tube to advance and retreat, and a syringe that is connected to the operation unit and applies negative pressure to the needle tube.

Further, the needle tube disclosed in WO2014/112518A comprises a slit that extends toward a proximal end side from a distal end opening thereof and the curvature radius of a first edge portion provided on both sides of the slit is adapted to be larger than the curvature radius of a second edge portion so that a step is formed between the first edge portion and the second edge portion.

According to the tissue collecting device disclosed in WO2014/112518A, the needle tube is inserted into lesional tissue and is then rotated to take biological tissue into the distal end portion of the tube from the gap of the step. Then, the needle tube is further rotated to excise the taken biological tissue by the edge portion of the slit. Accordingly, it is possible to collect the amount of biological tissue enough to make a pathological confirmation diagnosis.
WO 2019/157389 A1 discloses a needle for tissue collection in an endoscopic procedure. US 2018/228476 A1 discloses an endoscopic tri-point biopsy needle.

### SUMMARY OF THE INVENTION

However, the needle tube disclosed in WO2014/112518A (hereinafter, referred to as a biopsy needle) has a complicated structure that includes the first and second edge portions having curvature radii different from each other. For this reason, there is a problem that it is difficult to manufacture a small-diameter biopsy needle having an outer diameter of, for example, 3 mm or less.

Further, according to the tissue collecting device disclosed in WO2014/112518A, the biopsy needle is to be rotated about the axis of the biopsy needle in a case where biological tissue is to be taken in and excised by the biopsy needle. However, since the operation unit should be rotated together with the syringe in this case, an operator may be burdened.

The present invention has been made in consideration of the above-mentioned circumstances, and an object of the present invention is to provide a biopsy needle and a tissue collecting device that can collect the amount of biological tissue enough to make a pathological confirmation diagnosis with a simple structure and a simple operation.

In order to achieve the object of the present invention, a biopsy needle according to an aspect of the present invention is a biopsy needle that is to be inserted into an endoscope forceps channel. The biopsy needle comprises a hollow tube which includes a distal end opening portion and a proximal end opening portion and of which the distal end opening portion and the proximal end opening portion communicate with each other. The tube includes a puncture portion that is provided at a distal end portion of the tube and has at least one blade distal end point, a slit that extends toward the proximal end opening portion from the distal end opening portion, and a tissue excision cutter that is provided at a proximal end of the slit and is formed in a shape tapered toward a distal end of the slit from the proximal end of the slit. The puncture portion of the biopsy needle includes a first blade distal end point, a second blade distal end point, and a third blade distal end point. In a case where the tube is viewed in a direction orthogonal to an axial direction of the tube, the first blade distal end point is formed closer to a distal end side of the tube than the second blade distal end point and the third blade distal end point, and in a case where the tube is viewed in the axial direction of the tube, the first blade distal end point is disposed to face the slit in a radial direction of the tube and the second blade distal end point and the third blade distal end point are disposed to face each other with the slit interposed therebetween. The slit is an elongated through-hole that is formed in the shape of a line extending in the axial direction of the tube.

Further, in the biopsy needle according to the aspect of the present invention, it is preferable that the elongated through-hole is formed between a pair of wall portions facing each other in the direction orthogonal to the axial direction of the tube.

Further, in the biopsy needle according to the aspect of the present invention, it is preferable that the tissue excision cutter includes at least one inclined blade surface that is inclined to approach a central axis of the tube toward the distal end of the slit from the proximal end of the slit.

Furthermore, in the biopsy needle according to the aspect of the present invention, it is preferable that a sharp blade is formed on the inclined blade surface and the blade is formed at a position where the blade does not protrude to an outside of the tube from an outer peripheral surface of the tube.

Further, in the biopsy needle according to the aspect of the present invention, it is preferable that the inclined blade surface has a shape like a rounded chisel.

Moreover, in the biopsy needle according to the aspect of the present invention, it is preferable that the distal end portion of the tube includes a first blade surface that connects an outer peripheral surface of the tube to the first and second blade distal end points and has a normal line including a component toward an outside in the radial direction of the tube and a component toward the distal end side of the tube, and a second blade surface that connects the outer peripheral surface of the tube to the first and third blade distal end points and has a normal line including a component toward the outside in the radial direction of the tube and a component toward the distal end side of the tube.

Further, in the biopsy needle according to the aspect of the present invention, it is preferable that the at least one blade distal end point is formed on an inner peripheral surface of the tube.

Furthermore, in the biopsy needle according to the aspect of the present invention, it is preferable that a length between the first blade distal end point and a distal end of the tissue excision cutter in the axial direction of the tube is in a range of 0.5 mm to 25.0 mm.

Moreover, in the biopsy needle according to the aspect of the present invention, it is preferable that a width of the slit in a circumferential direction of the tube is smaller than an inner diameter of the tube.

Further, in the biopsy needle according to the aspect of the present invention, it is preferable that an outer diameter of the tube is in a range of 0.3 mm to 3.0 mm.

Furthermore, in the biopsy needle according to the aspect of the present invention, it is preferable that an inner diameter of the tube is in a range of 0.1 mm to 2.5 mm.

Moreover, in the biopsy needle according to the aspect of the present invention, it is preferable that an echo marker, which allows a position of the tube to be visually displayed in an ultrasound image, is provided on an outer peripheral surface of the tube.

Further, in the biopsy needle according to the aspect of the present invention, it is preferable that the echo marker is provided only around a periphery including the proximal end of the slit.

Furthermore, in the biopsy needle according to the aspect of the present invention, it is preferable that the echo marker is provided in a region extending toward a distal end side of the tube from a periphery, which includes the proximal end of the slit, as a starting point.

Moreover, in the biopsy needle according to the aspect of the present invention, it is preferable that the echo marker is provided in a region extending toward a proximal end side of the tube from a periphery, which includes the proximal end of the slit, as a starting point.

Further, in the biopsy needle according to the aspect of the present invention, it is preferable that the echo marker is formed in an uneven shape.

Furthermore, in the biopsy needle according to the aspect of the present invention, it is preferable that the tube is made of stainless steel, a nickel-titanium alloy, a nickel-chromium alloy, or a cobalt-chromium alloy.

In order to achieve the object of the present invention, a tissue collecting device according to another aspect of the present invention comprises the biopsy needle according to the aspect of the present invention and a suction member that communicates with the proximal end opening portion of the biopsy needle and generates negative pressure in the biopsy needle.

According to the present invention, it is possible to collect the amount of biological tissue enough to make a pathological confirmation diagnosis with a simple structure and a simple operation.

### BRIEF DESCRIPTION OF THE DRAWINGS

Fig. 1 is a cross-sectional view of a main part showing a state where a tissue collecting device according to an embodiment of the present invention is built in an ultrasonic endoscope.
Fig. 2 is a cross-sectional view showing the overall configuration of the tissue collecting device.
Fig. 3 is a cross-sectional view of an insertion unit of which a biopsy needle protrudes outward from a distal end opening of a sheath.
Fig. 4 is a cross-sectional view of the insertion unit of which the biopsy needle is retracted into the sheath.
Fig. 5 is an enlarged perspective view showing the configuration of a distal end portion of a tube of the tissue collecting device.
Fig. 6 is an enlarged perspective view showing the configuration of the distal end portion of the tube of the tissue collecting device.
Fig. 7 is a front view showing the configuration of the distal end portion of the tube of the tissue collecting device.
Fig. 8 is a diagram illustrating a method of collecting biological tissue using the tissue collecting device.
Fig. 9 is a perspective view of a distal end portion of a tube according to a first modification example.
Fig. 10 is a perspective view of a distal end portion of a tube according to a second modification example.
Fig. 11 is a perspective view of a distal end portion of a tube according to a third modification example.
Fig. 12 is a perspective view of a distal end portion of a tube according to a fourth modification example.
Fig. 13 is a perspective view of a distal end portion of a tube according to a fifth modification example.
Fig. 14 is a perspective view of a distal end portion of a tube according to a sixth modification example.
Fig. 15 is a perspective view of a distal end portion of a tube according to a seventh modification example.
Fig. 16 is a perspective view of a distal end portion of a tube according to an eighth modification example.
Fig. 17 is a perspective view showing a first aspect of an echo marker.
Fig. 18 is a perspective view showing a second aspect of the echo marker.
Fig. 19 is a perspective view showing a third aspect of the echo marker.
Fig. 20 is a perspective view showing a fourth aspect of the echo marker.

### DESCRIPTION OF THE PREFERRED EMBODIMENTS

Preferred embodiments of the present invention will be described in detail below with reference to the accompanying drawings.

A tissue collecting device for puncture to which the present invention is applied will be described as being adapted to be inserted into a body cavity through a treatment tool-insertion channel formed in an ultrasonic endoscope for performing electronic convex scanning, but a treatment tool-insertion channel of a scanning type ultrasonic endoscope or a normal endoscope, which does not comprise an ultrasound diagnosis mechanism, other than this ultrasonic endoscope, a trocar, and the like can be used as a guide unit of the tissue collecting device. In a case where the tissue collecting device is inserted into a trocar, the whole tissue collecting device can also be formed of a hard member.

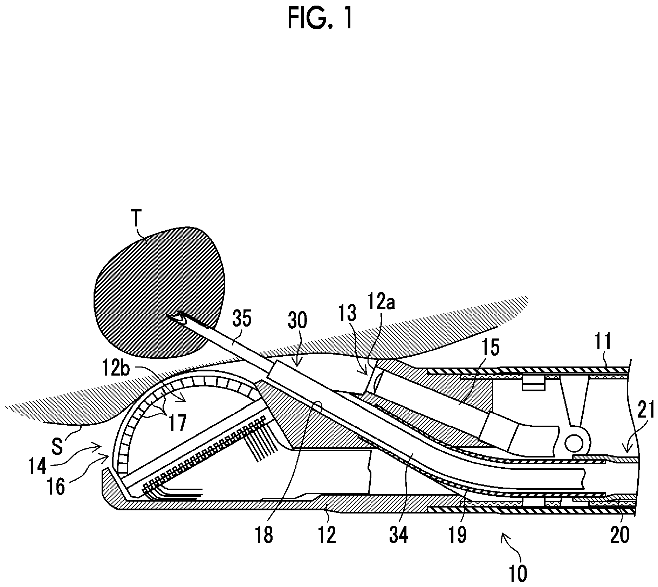

First, the configuration of a distal end portion of an ultrasonic endoscope that guides a tissue collecting device is shown in Fig. 1. In Fig. 1, reference numeral 10 denotes an insertion unit to be inserted into a body cavity, the insertion unit 10 is adapted so that a distal end portion body 12 is connected to a distal end of an angle part 11, and the distal end portion body 12 is provided with an endoscope observation part 13 on the proximal end side thereof and is provided with an ultrasonic observation part 14 on the distal end side thereof. The endoscope observation part 13 is provided in an inclined portion 12a provided on the proximal end side of the distal end portion body 12, and is adapted so that an observation field of view faces obliquely forward.

In Fig. 1, an illumination mechanism 15 comprising a light guide of the endoscope observation part 13 is shown and an observation mechanism is provided adjacent to this illumination mechanism 15 but this observation mechanism is not shown. A solid-state imaging element or an image guide can be used as the observation mechanism.

The ultrasonic observation part 14 includes an ultrasonic transducer unit 16 that is mounted on an opening portion 12b provided at the distal end of the distal end portion body 12. The ultrasonic transducer unit 16 is to perform electronic convex scanning, and includes a plurality of striped ultrasonic oscillators 17 that are arranged in an arc shape.

A treatment tool outlet portion 18 is formed at a position between the endoscope observation part 13 and the ultrasonic observation part 14. This treatment tool outlet portion 18 is a passage that is formed through the distal end portion body 12 and has a predetermined inner diameter, and a connection pipe 19 is connected to the treatment tool outlet portion 18. This connection pipe 19 is bent at a predetermined angle, and a flexible tube 20 is connected to the proximal end portion of the connection pipe 19. Accordingly, a treatment tool-insertion channel 21 includes the treatment tool outlet portion 18, the connection pipe 19, and the flexible tube 20, the treatment tool outlet portion 18 extends obliquely forward with respect to the axis of the insertion unit 10, the flexible tube 20 extends in the axial direction of the insertion unit 10, and the middle portion of the connection pipe 19 is bent by a predetermined angle.

Reference numeral 30 denotes a tissue collecting device according to an embodiment, and the tissue collecting device 30 is inserted into the treatment tool-insertion channel 21 and is adapted to be capable of appearing and disappearing from the treatment tool outlet portion 18. Further, the distal end portion body 12 is allowed to be in contact with an inner wall S of the body cavity, a biological tissue collection point T is positioned in the ultrasonic observation field of view by the ultrasonic observation part 14, a distal end portion of the tissue collecting device 30 is inserted into the inner wall S of the body cavity from the treatment tool outlet portion 18, and biological tissue can be collected after the distal end portion is guided up to the tissue collection point T.

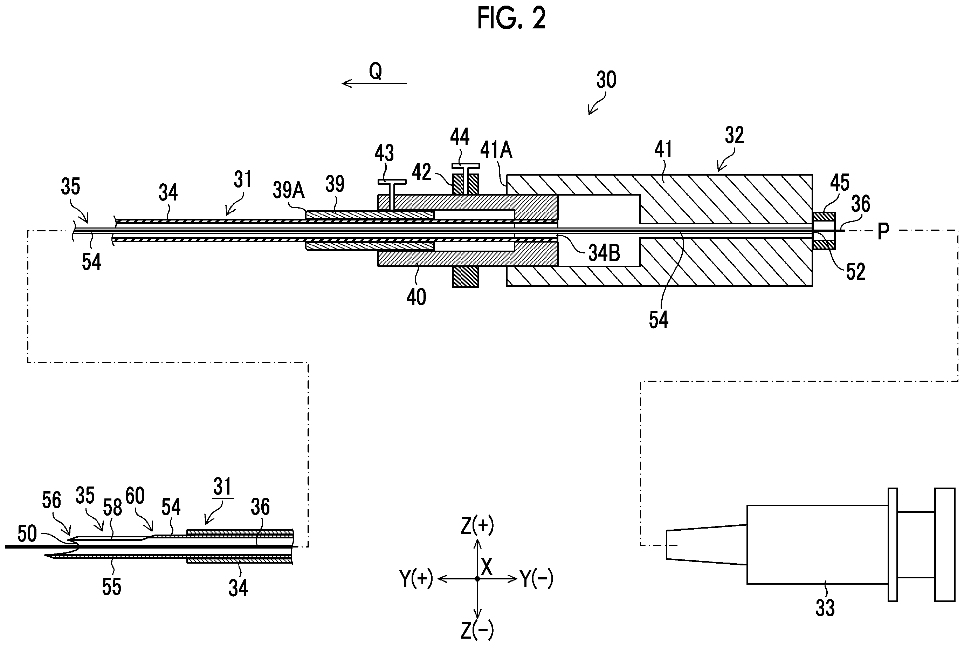

Fig. 2 is a cross-sectional view showing the overall configuration of the tissue collecting device 30. As apparent from Fig. 2, the tissue collecting device 30 includes an insertion unit 31 and an operation unit 32 and a syringe 33 is attachably and detachably connected to the proximal end portion of the operation unit 32. The syringe 33 is an example of a suction member.

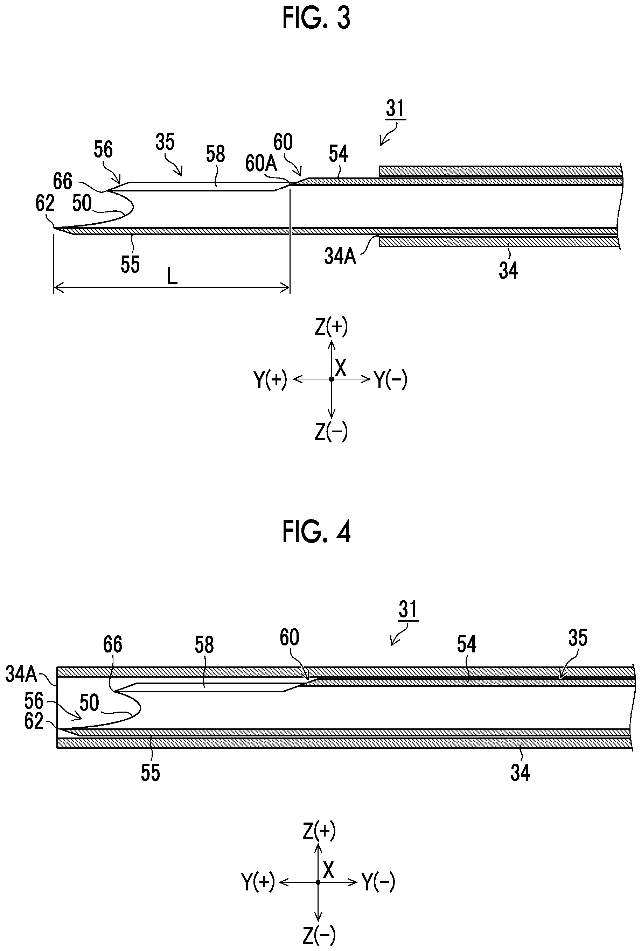

The insertion unit 31 has a length longer than the total length of at least the treatment tool-insertion channel 21, and is formed of a double tubular member as shown in Figs. 3 and 4. That is, the insertion unit 31 includes a sheath 34 and a biopsy needle 35 inserted into the sheath 34 that are arranged in this order from the outermost peripheral side. Further, Fig. 3 is a cross-sectional view of the insertion unit 31 showing a state where the biopsy needle 35 protrudes outward from a distal end opening 34A of the sheath 34, and Fig. 4 is a cross-sectional view of the insertion unit 31 showing a state where the biopsy needle 35 is retracted into the sheath 34.

The sheath 34 is inserted into the treatment tool-insertion channel 21 of an endoscope and forms the exterior of the insertion unit 31. The sheath 34 consists of a tubular member having flexibility, and is formed of a resin member made of, for example, polyethersulfone, Teflon (registered trademark), or polyetheretherketone (PEEK). The sheath 34 may be formed of a contact coil or the like.

The biopsy needle 35 is used to puncture biological tissue to collect lesional tissue and the like, and is inserted into the sheath 34 to freely advance and retreat. The detailed configuration of the biopsy needle 35 will be described later, but the biopsy needle 35 includes a tube 54. The tube 54 includes a distal end opening portion 50 and a proximal end opening portion 52 (see Fig. 2), and the distal end opening portion 50 and the proximal end opening portion 52 communicate with each other. Further, as shown in Figs. 3 and 4, a distal end portion 55 of the tube 54 is provided with a puncture portion 56, a slit 58, and a tissue excision cutter 60.

Since the tube 54 is to be inserted into the body, the tube should be hard. Further, since the tube 54 is to be inserted into the treatment tool-insertion channel 21, the tube 54 should have flexibility in a bending direction so that the tube 54 can pass through the bent connection pipe 19 and be smoothly inserted into the angle part 11 even in a state where the angle part 11 is curved. For this reason, the tube 54 is made of stainless steel, a nickel-titanium alloy, a nickel-chromium alloy, or a cobalt-chromium alloy having a small diameter and flexibility. Stainless steel is hard to rust, nickel-titanium alloy hardly has a bending habit even though being inserted into the bent connection pipe 19 since being a superelastic alloy, and nickel-chromium alloy and cobalt-chromium alloy can be easily inserted into the biological tissue collection point T since having a high hardness. At least the distal end portion 55, which includes the puncture portion 56, of the biopsy needle 35 may be formed of a hard pipe, and a portion of the biopsy needle 35 other than the distal end portion 55 may be formed of a soft tube having flexibility.

The tube 54 is inserted into and disposed in the sheath 34 to freely advance and retreat, and is moved to a retreat position (a position shown in Fig. 4) where the tube 54 is retracted into the sheath 34 and an operating position (a position shown in Fig. 3) where the tube 54 protrudes from the distal end opening 34A of the sheath 34 by a predetermined length.

For this purpose, the proximal end portion of the tube 54 is connected to the operation unit 32 and the tube 54 is adapted to appear and disappear from the distal end opening 34A of the sheath 34 by the operation of the operation unit 32. The specific configuration of the operation unit 32 is shown in Fig. 2.

As shown in Fig. 2, the operation unit 32 includes a luer lock-operation part 39, a sheath position-operation part 40, a tube position-operation part 41, and a stopper ring 42. Further, a stylet 36 is inserted into the tube 54.

A luer lock portion 39A is formed at the distal end portion of the luer lock-operation part 39, and the luer lock portion 39Ais used to be fixed to a treatment tool inlet port (not shown) of the ultrasonic endoscope and is attachably and detachably connected to, for example, a pair of luer lock portions that are present at the treatment tool inlet port. The luer lock-operation part 39 is inserted into a hole portion of the sheath position-operation part 40, and the luer lock-operation part 39 and the sheath position-operation part 40 are mounted to be relatively slidable along an axis P of the operation unit 32. The luer lock-operation part 39 and the sheath position-operation part 40 are fixed to each other by a screw 43 that is screwed toward the outer peripheral surface of the luer lock-operation part 39 from the outer peripheral surface of the sheath position-operation part 40.

Further, the stopper ring 42 is provided on the outer peripheral surface of the sheath position-operation part 40 to be slidable along the axis P, and the stopper ring 42 is fixed by a screw 44 that is screwed toward the outer peripheral surface of the sheath position-operation part 40 from the outer peripheral surface of the stopper ring 42. The sheath position-operation part 40 is inserted into a hole portion of the tube position-operation part 41, and the sheath position-operation part 40 and the tube position-operation part 41 are mounted to be relatively slidable along the axis P. The relative positions of the sheath position-operation part 40 and the tube position-operation part 41 in the direction of the axis P are determined in a case where a distal end surface 41A of the tube position-operation part 41 is in contact with the stopper ring 42.

In the operation unit 32 having the above-mentioned configuration, with regard to the sheath 34, the proximal portion side of the sheath 34 is inserted into the hole portion of the sheath position-operation part 40 from the hole portion of the luer lock-operation part 39 and is fixed to the proximal end portion of the sheath position-operation part 40. Accordingly, a proximal end opening 34B of the sheath 34 is opened to the proximal end portion of the sheath position-operation part 40. Further, with regard to the tube 54, the proximal end side of the tube 54 is inserted into the hole portion of the tube position-operation part 41 from the proximal end opening 34B of the sheath 34 and is fixed to the proximal end portion of the tube position-operation part 41. Therefore, a proximal end opening portion 52 of the tube 54 is opened to the proximal end portion of the tube position-operation part 41. Furthermore, the proximal end portion of the tube position-operation part 41 is provided with a luer lock portion 45 communicating with the proximal end opening portion 52, and the stylet 36 is provided between the luer lock portion 45 and a position protruding from the tube distal end opening portion 50. The stylet 36 functions to protect blade distal end points 62, 64, and 66, to protect the sheath 34 from the blade distal end points 62, 64, and 66, and to suppress the entrance of foreign substances into the tube 54. In a case where the stylet 36 protrudes from the tube distal end opening portion 50 and the sheath 34 is bent in a curved shape to form a part of an arc, the distal end surface of the stylet 36 and the outer peripheral surface of the tube distal end opening portion are in contact with the inner surface of the sheath 34. In addition, the distal end surface of the stylet 36 is pressed from the inner surface of the sheath 34, so that the tube distal end opening portion 50 is in a state where the distal end opening portion 50 retreats to a position away from the inner surface of the sheath 34. In a case where the stylet 36 is completely removed from the luer lock portion 45 in a proximal direction, the syringe 33, which includes a pair of luer locks for suction and liquid pumping, can be detached from the luer lock portion 45.

Next, the usage example of the tissue collecting device 30 shown in Fig. 2 will be described.

First, the sheath 34 into which the tube 54 is inserted is inserted into the treatment tool-insertion channel 21 shown in Fig. 1 from the treatment tool inlet port of the ultrasonic endoscope, and the luer lock portion 39A of the luer lock-operation part 39 is fixed to a pair of luer locks of the treatment tool inlet port in a case where, for example, the distal end portion of the sheath 34 is positioned near the distal end of the treatment tool outlet portion 18 shown in Fig. 1.

Then, the screw 43 is loosened, and the sheath position-operation part 40 is allowed to slide with respect to the luer lock-operation part 39 in the direction of an arrow Q along the axis P. Accordingly, the distal end portion of the sheath 34 protrudes from the treatment tool outlet portion 18. After that, the slide position of the sheath position-operation part 40 is adjusted, and the sheath position-operation part 40 is fixed to the luer lock-operation part 39 by the screw 43 in a case where the protruding length of the sheath 34 is an appropriate length.

Then, the screw 44 is loosened, the stopper ring 42 is allowed to slide with respect to the sheath position-operation part 40 along the axis P, and the position of the stopper ring 42 with respect to the sheath position-operation part 40 is adjusted. That is, the protruding length of the tube 54 from the distal end portion of the sheath 34 is adjusted. A user can make this adjustment while looking at, for example, gradations printed on the outer peripheral surface of the sheath position-operation part 40. After that, the stopper ring 42 is fixed to the sheath position-operation part 40 by the screw 44.

Then, the stylet 36 protruding from the distal end opening portion 50 is slightly pulled toward the proximal end side and is retracted from the distal end opening portion 50 of the tube by a length in a range of about 5 mm to 10 mm. Accordingly, the blade distal end points 62, 64, and 66 are likely to be inserted into the biological tissue collection point T.

After that, the tube position-operation part 41 is allowed to slide with respect to the sheath position-operation part 40 in the direction of the arrow Q along the axis P. The distal end portion 55 of the tube 54 protrudes from the distal end opening 34A of the sheath 34 due to this slide operation, and the distal end portion 55 of the tube 54 is inserted into the biological tissue collection point T (see Fig. 1) in a case where the distal end surface 41A of the tube position-operation part 41 is in contact with the stopper ring 42.

Then, the stylet 36 is pulled out from the luer lock portion 45 and is completely removed. After that, the syringe 33 is mounted on the luer lock portion 45 and suction can be performed. The above is the usage example of the tissue collecting device 30.

Further, the tube 54 also functions as a fluid passage. This fluid passage acts as a suction passage that applies a negative pressure and a passage that pumps normal saline to discharge biological tissue collected in the tube 54.

Next, the configuration of the distal end portion 55 of the tube 54 will be described.

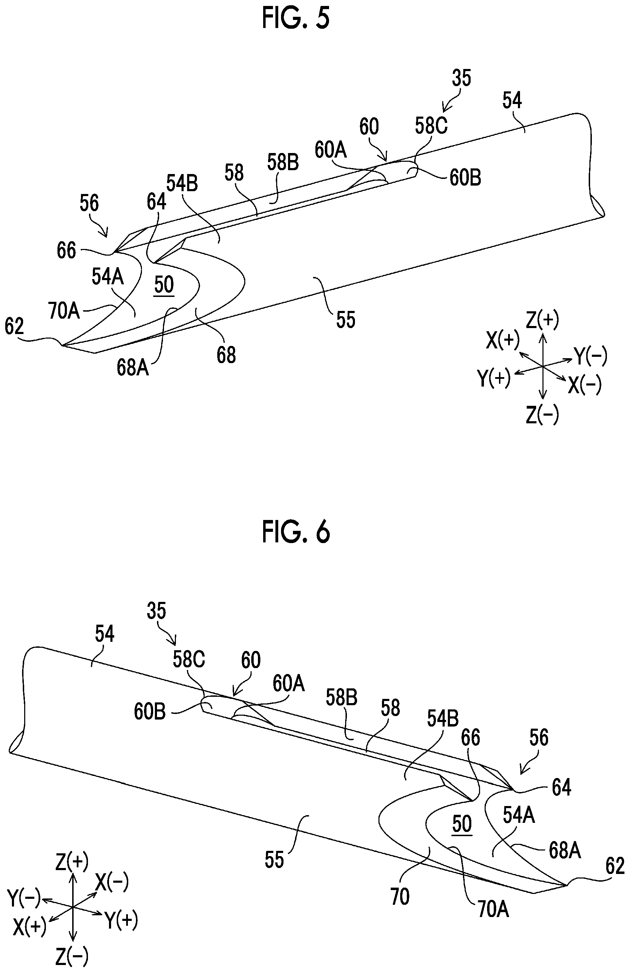

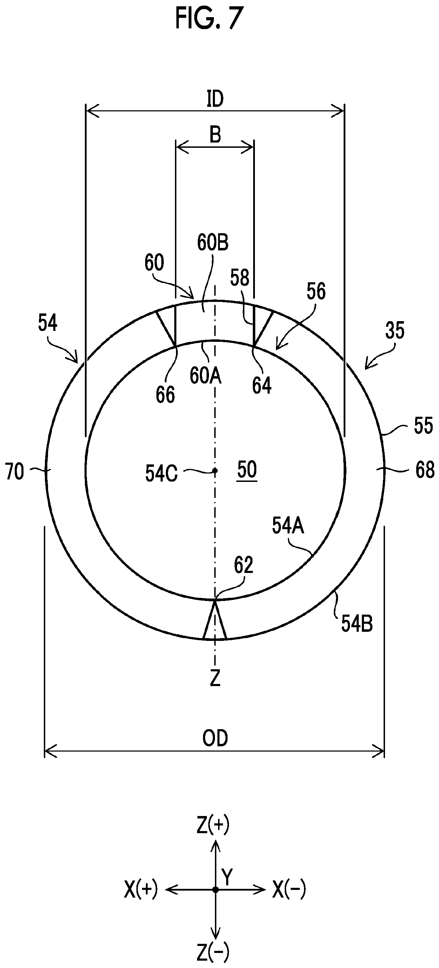

Fig. 5 is an enlarged perspective view showing the configuration of the distal end portion 55 of the tube 54, and is a perspective view of one side surface of the distal end portion 55 viewed from the distal end side of the tube 54. Fig. 6 is a perspective view of the other side surface of the distal end portion 55 viewed from the distal end side of the tube 54. Further, Fig. 7 is a front view of the distal end portion 55 viewed from the distal end side of the tube 54.

A description will be made below using a three dimensional Cartesian coordinate system of an X axis, a Y axis, and a Z axis the description of the configuration of the distal end portion 55 of the tube 54. That is, in a case where a direction where the slit 58 faces is defined as an upward direction as the distal end portion 55 of the tube 54 is viewed from the operation unit 32 (see Fig. 2), the upward direction is referred to as a Z(+) direction and a downward direction, which is a direction opposite to the upward direction, is referred to as a Z(-) direction. Further, a right direction in this case is referred to as an X(+) direction and a left direction is referred to as an X(-) direction. Furthermore, a direction toward the distal end side in this case is referred to as a Y(+) direction and a direction toward the proximal end side is referred to as a Y(-) direction.

As shown in Figs. 5 to 7, the distal end portion 55 of the tube 54 includes the puncture portion 56, the slit 58, and the tissue excision cutter 60 described above.

As shown in Fig. 5, the slit 58 is an elongated through-hole that is formed in the shape of a line extending in the Y(-) direction toward the proximal end opening portion 52 (see Fig. 2) from the distal end opening portion 50, and functions as an intake port that is used to take a large amount of biological tissue into the tube 54 from the side surface of the distal end portion 55 during the collection of tissue. The slit 58 is formed of a gap between a pair of wall portions 58A and 58B facing each other in the X direction and extending in the Y direction.

The tissue excision cutter 60 is provided at a proximal end 58C of the slit 58. The tissue excision cutter 60 is formed in a shape tapered toward the distal end of the slit 58 (Y(+) side) from the proximal end 58C. Further, the tissue excision cutter 60 includes an inclined blade surface 60B that is inclined to approach a central axis 54C of the tube 54 toward the distal end of the slit 58 (Y(+) side) from the proximal end 58C of the slit 58. A sharp blade 60Ais formed toward the distal end of the slit 58 (Y(+) side) on the inclined blade surface 60B, and this blade 60A is formed on an inner peripheral surface 54A of the tube 54. Since this tissue excision cutter 60 is provided at the proximal end 58C of the slit 58, biological tissue taken into the distal end portion 55 through the slit 58 can be excised by the operation of the tube 54 in the Y(+) direction, that is, the operation of the tube 54 in the same direction as a puncture direction with respect to the biological tissue collection point T. Since the tissue excision cutter 60 may be formed in a tapered shape as described above, the blade 60A does not necessarily need to be sharp and has only to be thin enough to have a cutter function.

The puncture portion 56 is a portion that is to be inserted into the biological tissue collection point T (see Fig. 1) from the inner wall S of the body cavity (see Fig. 1) in a case where the tube 54 is operated in the Y(+) direction, and is a portion that holds the biological tissue collection point T. The puncture portion 56 of this example includes, for example, the blade distal end point 62, the blade distal end point 64, and the blade distal end point 66. Each of these blade distal end points 62, 64, and 66 is sharply formed so that distal ends thereof gradually become thinner toward the Y(+) direction. Since the puncture portion 56 includes the plurality of blade distal end points 62, 64, and 66 as described above, the biological tissue collection point T can be reliably held inside the tube 54 without escaping to the outside of the tube 54. The blade distal end point 62 is an example of a first blade distal end point, the blade distal end point 64 is an example of a second blade distal end point, and the blade distal end point 66 is an example of a third blade distal end point.

Each of the blade distal end points 62, 64, and 66 is formed on the inner peripheral surface 54A of the tube 54. The inner peripheral surface 54A refers to a surface of which the normal line direction faces the central axis 54C of the tube 54 in a plane (Z-X plane) orthogonal to the axial direction (Y direction) of the tube 54. Since the respective blade distal end points 62, 64, and 66 are formed on this inner peripheral surface 54A, the blade distal end points 62, 64, and 66 are formed at positions overlapping with the inner peripheral surface 54A in a case where the blade distal end points 62, 64, and 66 are viewed in the axial direction of the tube 54 (Y direction). Accordingly, in a case where the tube 54 is inserted into and removed from the sheath 34, the blade distal end points 62, 64, and 66 do not come in contact with the inner surface of the sheath 34 and damage to the sheath 34 can be prevented.

Further, in a case where the tube 54 is viewed in a direction (X direction) orthogonal to the axial direction of the tube 54 (Y direction), the blade distal end point 62 is formed closer to the distal end side of the tube 54 than the blade distal end points 64 and 66. Furthermore, in a case where the tube 54 is viewed in the axial direction of the tube 54 (Y direction), the blade distal end point 62 is disposed to face the slit 58 in the radial direction of the tube 54 (Z direction) and the blade distal end points 64 and 66 are disposed to face each other in the X direction with the slit 58 interposed therebetween. In a case where the tube 54 is viewed in the direction (X direction) orthogonal to the axial direction of the tube 54 (Y direction), the blade distal end points 64 and 66 are formed at the same position in the axial direction of the tube 54 (Y direction). However, this is an example, and the blade distal end points 64 and 66 may be formed at positions shifted from each other in the axial direction of the tube 54 (Y direction).

Such these blade distal end points 62, 64, and 66 are formed at the distal end portion 55, so that two blade surfaces 68 and 70 are formed at the distal end portion 55 of the tube 54.

That is, as shown in Fig. 5, the blade surface 68 is formed as a surface that connects an outer peripheral surface 54B of the tube 54 to the blade distal end points 62 and 64. The blade surface 68 has a normal line that includes a component toward the outside in the radial direction of the tube 54 (X(-) direction) and a component toward the distal end side of the tube 54 (Y(+) side). Since this blade surface 68 is formed, a sharp blade 68A is formed at a portion where the blade surface 68 and the inner peripheral surface 54A intersect with each other. This blade surface 68 is an example of a first blade surface.

As shown in Fig. 6, the blade surface 70 is formed as a surface that connects the outer peripheral surface 54B of the tube 54 to the blade distal end points 62 and 66. The blade surface 70 has a normal line that includes a component toward the outside in the radial direction of the tube 54 (X(+) direction) and a component toward the distal end side of the tube 54 (Y(+) side). Since this blade surface 70 is formed, a sharp blade 70A is formed at a portion where the blade surface 70 and the inner peripheral surface 54A intersect with each other. This blade surface 70 is an example of a second blade surface.

The above is the configuration of the distal end portion 55 of the tube 54. As shown in Fig. 7, the tube 54 of the embodiment is formed in a shape symmetric with respect to a Z axis, which passes through the central axis 54C of the tube 54, as the axis of symmetry, but is not limited to this shape. The tube 54 may be formed in an asymmetric shape.

The embodiment is configured as described above. Next, an example of a method of collecting biological tissue using this tissue collecting device 30 will be described.

First, as shown in Fig. 1, the distal end portion body 12 of the ultrasonic endoscope is disposed at a predetermined position on the inner wall S of the body cavity. In this state, in a case where the tissue collection point T in the body is captured in the observation field of view of the ultrasonic transducer unit 16 included in the ultrasonic observation part 14, the insertion unit 31 of the tissue collecting device 30 is inserted into the treatment tool-insertion channel 21. In this step, the sheath distal end opening 34A is in a position where the distal end opening 34A of the sheath does not protrude from the distal end of the treatment tool outlet portion 18.

Then, the sheath position-operation part 40 is operated in the direction of the arrow Q to make the sheath distal end opening 34A protrude from the distal end of the treatment tool outlet portion 18, and the position of the sheath position-operation part 40 is fixed at a position appropriate for puncture by the screw 43.

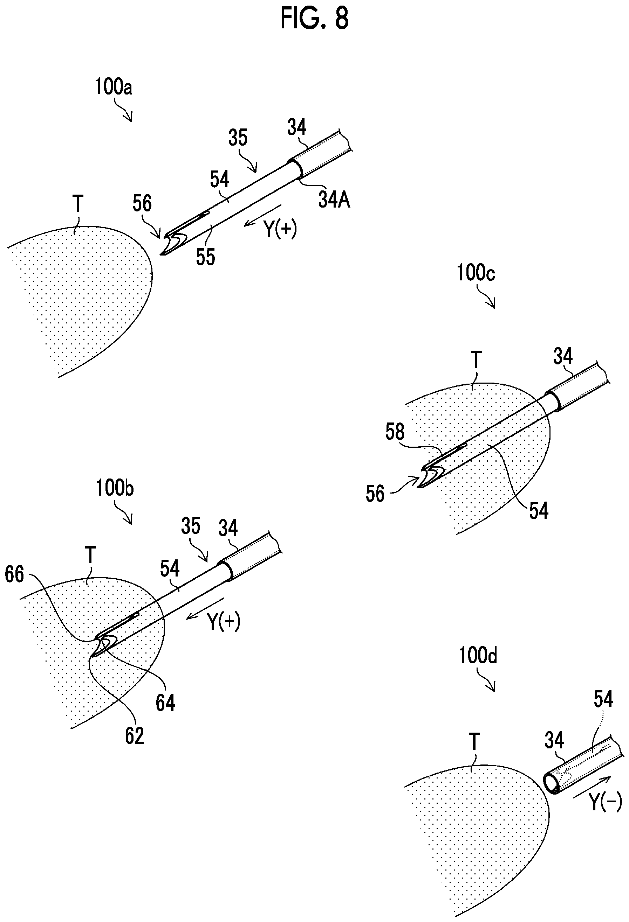

Here, in a state where the insertion unit 31 is not yet inserted into the inner wall S of the body cavity, the distal end portion 55 of the tube 54 of the insertion unit 31 is covered with the sheath 34 (see Fig. 4). In a case where the tube position-operation part 41 (see Fig. 2) is operated in the direction of the arrow Q in this state, the distal end portion 55 of the tube 54 protrudes in the Y(+) direction from the distal end opening 34A of the sheath 34 as shown in a portion 100a of Fig. 8. Then, due to the continuous protruding operation of the tube 54 in the Y(+) direction, the puncture portion 56 of the distal end portion 55 is inserted into the tissue collection point T from the inner wall S of the body cavity (see Fig. 1) as shown in a portion 100b of Fig. 8.

At this time, the stylet 36 (see Fig. 2) is retracted from the distal end opening portion 50 by a length in a range of about 5 mm to 10 mm at the time of 100a of Fig. 8 and the tube distal end portion 55 is then allowed to protrude, so that the blade distal end points can be inserted into the tissue collection point T. Further, after 100b of Fig. 8, the stylet 36 is allowed to protrude from the tube distal end opening portion 50 again, so that a stomach wall and other foreign substances mixed in at the time of insertion can be discharged to the outside of the tube. Accordingly, only a specimen more appropriate for pathological diagnosis can be obtained.

Further, the blade distal end point 62, which is positioned closest to the distal end side, among the blade distal end points 62, 64, and 66 functions as a blade edge that forms the starting point of insertion. Furthermore, the blade distal end points 64 and 66 following the blade distal end point 62 are smoothly inserted into the tissue collection point T from the inner wall S of the body cavity while being guided by the blade surfaces 68 and 70 preceding the blade distal end points 64 and 66. Here, since the puncture portion 56 includes the plurality of blade distal end points 62, 64, and 66 in the embodiment, the tissue collection point T into which the puncture portion 56 is inserted can be reliably held inside the tube 54 without escaping to the outside of the tube 54. Further, since the insertion path of the tube 54 at this time is captured in the ultrasonic observation field of view, it is possible to safely perform an operation for inserting the tube 54 and to reliably hit the tissue collection point T.

After that, in a case where the puncture portion 56 is inserted into the biological tissue (for example, a tumor) of the biological tissue collection point T as shown in a portion 100c of Fig. 8 due to the continuous protruding operation of the tube 54 in the Y(+) direction, a part of the biological tissue enters the tube 54 from the slit 58. Then, in a case where the tube 54 further protrudes in the Y(+) direction in this state, the biological tissue entering the tube 54 is excised by the blade 60A of the tissue excision cutter 60 and the excised biological tissue is collected in the tube 54. Accordingly, the amount of biological tissue enough to make a pathological confirmation diagnosis is collected by the tube 54.

It is possible to obtain larger biological tissue by applying a suction force to the above-mentioned procedure. There are mainly following two types of methods of applying a suction force. The first method is a method using the syringe 33. In a case where the stylet 36 is completely removed from the luer lock portion 45 in the proximal direction after 100b of Fig. 8, the syringe 33 is then mounted on the luer lock portion 45, and the inside of the tube 54 is maintained under negative pressure by the operation of the syringe 33, a suction force is generated in the slit 58. Accordingly, the amount of biological tissue entering the slit 58 is further increased. Further, in a case where the tube 54 is reciprocated in the biological tissue in a state where a suction force is generated in the slit 58, the biological tissue continuously enters the tube 54 through the slit 58. Accordingly, more biological tissue can be efficiently collected.

The second suction method is a method using the stylet 36. In a case where 100c of Fig. 8 is performed while the stylet 36 is slowly removed in the proximal direction of the luer lock portion 45, slight negative pressure can be applied to the inside of the tube 54. This also makes it possible to obtain large biological tissue.

After biological tissue is collected by the insertion unit 31 of the tissue collecting device 30 as described above, the insertion unit 31 is taken out from the treatment tool-insertion channel 21. In this case, for example, after the tube 54 is moved in the Y(-) direction to retract the distal end portion 55 of the tube 54 into the sheath 34 as shown in a portion 100d of Fig. 8, the insertion unit 31 may be taken out from the treatment tool-insertion channel 21 in this state.

After the insertion unit 31 is taken out from the treatment tool-insertion channel 21, for example, a syringe for pumping normal saline is connected to the luer lock portion 45 instead of the syringe 33 for suction and normal saline is pumped into the tube 54 from this syringe. Accordingly, the collected biological tissue can be transferred to a test tube or the like. Alternatively, the stylet 36 can also be reinserted from the luer lock portion 45 and be allowed to protrude up to the tube distal end opening portion 50 to push out and discharge the biological tissue present in the tube.

According to the embodiment, as described above, the tube 54 including the puncture portion 56 and the slit 58 employs a configuration in which the tissue excision cutter 60 is provided at the proximal end 58C of the slit 58. Accordingly, it is possible to collect the amount of biological tissue enough to make a pathological confirmation diagnosis with a simple structure. Further, according to the above-mentioned configuration, it is possible to collect biological tissue by an operation for moving the tube 54 in the Y(+) direction (the operation of the tube 54 in the same direction as a puncture direction with respect to biological tissue). Accordingly, it is possible to collect biological tissue by a simple operation as compared to the tissue collecting device disclosed in WO2014/112518A of which the tube is to be rotated at the time of collection of biological tissue.

Therefore, according to the embodiment, it is possible to collect the amount of biological tissue enough to make a pathological confirmation diagnosis with a simple structure and a simple operation.

Further, according to the embodiment, since the blade 60A of the tissue excision cutter 60 is formed on the inner peripheral surface 54A of the tube 54, the blade 60A does not pierce or is not caught by the inner peripheral surface of the sheath 34 or the distal end portion body 12 and the tube 54 can be smoothly operated to advance and retreat in the sheath 34.

An example in which the blade 60A is formed on the inner peripheral surface 54A of the tube 54 has been described in the embodiment, but the blade 60A may be formed, for example, between the inner peripheral surface 54A and the outer peripheral surface 54B or may be formed on the same surface as the outer peripheral surface 54B. Further, the blade 60Amay be formed at a position where the blade protrudes to the outside of the tube 54 from the outer peripheral surface 54B. However, in terms of preventing the above-mentioned piercing and catching, it is preferable that the blade 60A is formed at a position where the blade does not protrude to the outside of the tube 54 from the outer peripheral surface 54B of the tube 54.

Furthermore, since the tissue excision cutter 60 includes the inclined blade surface 60B in the embodiment, the biological tissue excised by the blade 60A can be reliably cut from the tissue collection point T by the inclined blade surface 60B.

Further, since the puncture portion 56 includes the plurality of blade distal end points 62, 64, and 66 in the embodiment, the puncture portion 56 is easily inserted into the biological tissue collection point T and the biological tissue collection point T into which the puncture portion 56 is inserted can be reliably held without escaping to the outside of the tube 54. The three blade distal end points 62, 64, and 66 have been exemplified in the embodiment, but the puncture portion 56 has only to include at least one blade distal end point. However, it is preferable in terms of the above-mentioned insertion and holding that the puncture portion 56 includes a plurality of blade distal end points.

Furthermore, a configuration in which the blade distal end point 62 farthest from the slit 58 in the Y direction and the slit 58 are disposed to face each other in the radial direction of the tube 54 (Z direction) has been employed in the embodiment. That is, since a configuration in which the slit 58 comes into contact with biological tissue after the blade distal end point 62 is sufficiently inserted into the biological tissue collection point T has been employed, the biological tissue can be appropriately collected.

Further, a configuration in which the blade surfaces 68 and 70 are formed on the distal end portion 55 of the tube 54 has been employed in the embodiment. That is, since a configuration in which the inner peripheral surface 54A of the tube 54 always comes into contact with the biological tissue collection point T prior to the outer peripheral surface 54B of the tube 54 is employed, the biological tissue can be appropriately collected.

Furthermore, it is preferable that a length L between the blade distal end point 62 and the blade 60A of the tissue excision cutter 60 in the axial direction of the tube 54 (Y direction) is in a range of 0.5 mm to 25.0 mm as shown in Fig. 3. Accordingly, since the starting point of insertion can be reliably formed by the blade distal end point 62, and the slit 58 can be easily inserted into the biological tissue collection point T by the total length thereof during the procedure, the biological tissue can be appropriately collected.

Further, it is preferable that a width B of the slit 58 in the circumferential direction of the tube 54 is smaller than the inner diameter ID of the tube 54 as shown in Fig. 7. Accordingly, since it is difficult for biological tissue, which is taken into the distal end portion 55, to escape to the outside of the distal end portion 55, the biological tissue can be appropriately collected.

Furthermore, it is preferable that the outer diameter OD of the tube 54 is in a range of 0.3 mm to 3.0 mm as shown in Fig. 7. Accordingly, the tube 54 can be satisfactorily inserted into the sheath 34 and the degree of invasion to a patient can be suppressed.

Further, it is preferable that the inner diameter ID of the tube 54 is in a range of 0.1 mm to 2.5 mm as shown in Fig. 7. Accordingly, a large amount of biological tissue can be taken into the tube 54 while the strength of the tube 54 is maintained.

The biopsy needle and the tissue collecting device according to the embodiment of the present invention have been described in detail above, but it is natural that the present invention is not limited to the above-mentioned examples and may have various improvements and modifications without departing from the scope of the present invention. Some modification examples will be described below. In the description of the following modification examples, the same members as or members similar to those of the tube 54 shown in Figs. 3 to 7 will be denoted by the same reference numerals as those of the tube 54.

### [First modification example]

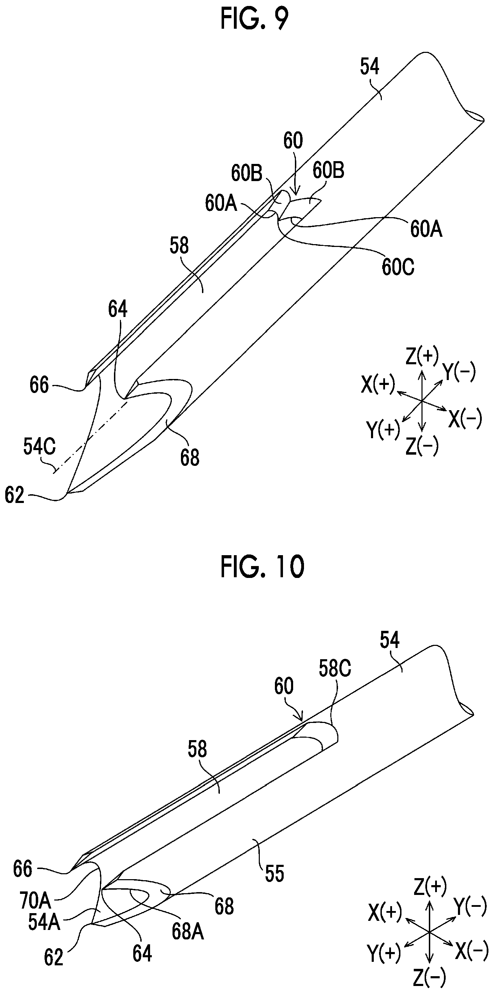

In a first modification example shown in Fig. 9, a blade distal end point 60C is formed on the blade 60A of the tissue excision cutter 60. The blade distal end point 60C is formed in the middle portion of the blade 60A in the circumferential direction of the tube 54 and is formed to be sharp toward the distal end of the slit 58.

According to the first modification example, since the blade distal end point 60C functions as a blade edge that forms the starting point of insertion on biological tissue entering the tube 54 through the slit 58, the biological tissue can be smoothly excised by the blade 60A following the blade distal end point 60C. The tissue excision cutter 60 shown in Fig. 9 includes two inclined blade surfaces 60B and 60B connected on both sides in the direction of the X axis with the blade distal end point 60C interposed therebetween, but may include at least one inclined blade surface 60B.

### [Second modification example]

In a second modification example shown in Fig. 10 and not falling under the scope of protection of the claims, the blade distal end point 62 is formed at the same position as the blade distal end points 64 and 66 in the axial direction of the tube 54 (Y direction). Other configuration is the same as that of the tube 54 shown in Figs. 6 to 8.

According to the second modification example, the blade distal end points 62, 64, and 66 are simultaneously inserted into the biological tissue collection point T, but the tissue collection point T into which the blade distal end points 62, 64, and 66 are inserted can be reliably held inside the tube 54 without escaping to the outside of the tube 54 as in the tube 54 shown in Figs. 6 to 8. The position of the blade distal end point 62 is not limited to the above-mentioned position, and the blade distal end point 62 may be formed closer to the proximal end side of the tube 54 than the blade distal end points 64 and 66 as long as it is possible to prevent the tissue collection point T from escaping to the outside of the tube 54.

### [Third modification example]

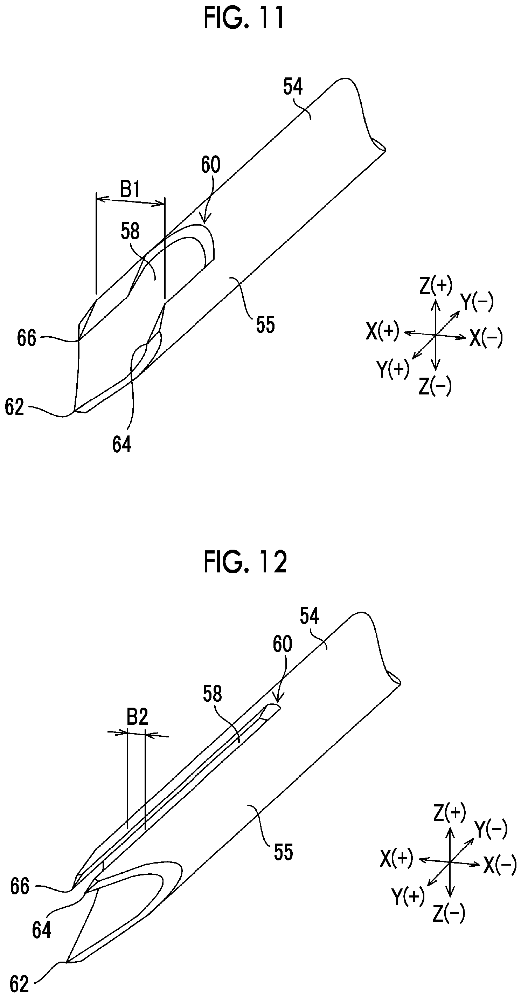

In a third modification example shown in Fig. 11 and not falling under the scope of protection of the claims, a width B1 of the slit 58 is larger than the width B shown in Fig. 7. Further, the width B1 is smaller than the inner diameter ID (see Fig. 7) of the tube 54.

According to the third modification example, since the width B1 of the slit 58 is large, more biological tissue can be allowed to enter the distal end portion 55.

### [Fourth modification example]

In a fourth modification example shown in Fig. 12, a width B2 of the slit 58 is smaller than the width B shown in Fig. 7. Further, the width B2 is smaller than the inner diameter ID (see Fig. 7) of the tube 54.

According to the fourth modification example, since a suction force larger than that in the case of the width B is generated in the slit 58 in a case where the syringe 33 is operated, a sufficient amount of biological tissue can be allowed to enter the distal end portion 55.

### [Fifth modification example]

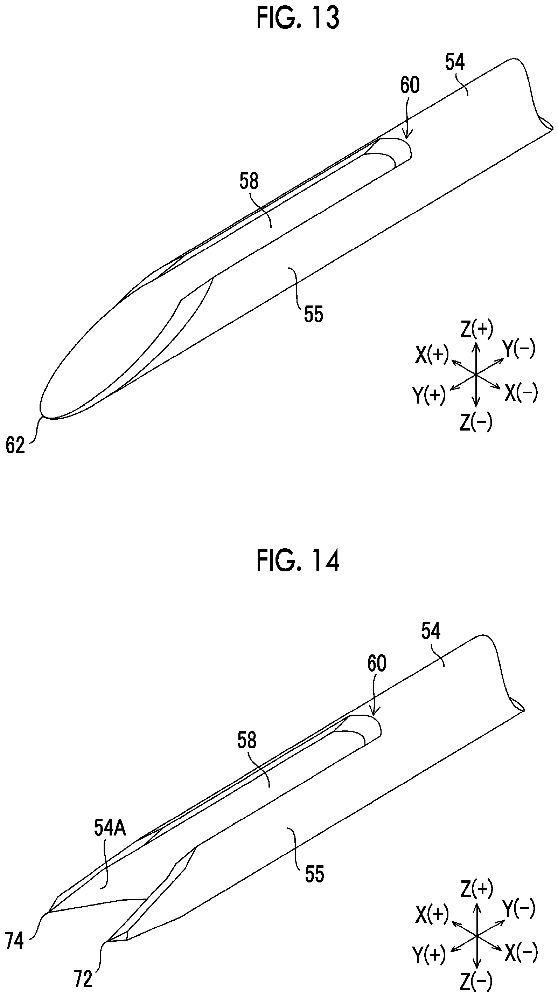

In a fifth modification example shown in Fig. 13 and not falling under the scope of protection of the claims, among the three blade distal end points 62, 64, and 66 shown in Figs. 5 to 7, only the blade distal end point 62 formed closest to the distal end side is formed on the distal end portion 55. This blade distal end point 62 is disposed to face the slit 58 in the radial direction of the tube 54 as described above.

According to the fifth modification example, in a case where the blade distal end point 62 is inserted into the biological tissue collection point T, the biological tissue collection point T can be held by the blade distal end point 62 and biological tissue can be allowed to enter the distal end portion 55 through the slit 58.

### [Sixth modification example]

In a sixth modification example shown in Fig. 14 and not falling under the scope of protection of the claims, two blade distal end points 72 and 74 are formed at the distal end portion 55 of the tube 54. The blade distal end points 72 and 74 are formed on the extension lines of the inner peripheral surface 54A of the tube 54 in the Y(+) direction, respectively. Further, in a case where the tube 54 is viewed in a direction (X direction) orthogonal to the axial direction of the tube 54 (Y direction), the blade distal end points 72 and 74 are formed at the same position in the axial direction of the tube 54 (Y direction). Furthermore, in a case where the tube 54 is viewed in the axial direction of the tube 54 (Y direction), the blade distal end points 72 and 74 are disposed to face each other in the radial direction of the tube 54 (Z direction) but are disposed not to face the slit 58 and are formed to have a phase difference of 90° in the circumferential direction.

According to the sixth modification example, in a case where the blade distal end points 72 and 74 are inserted into the biological tissue collection point T, the biological tissue collection point T can be reliably held by the blade distal end points 72 and 74 and biological tissue can be allowed to enter the distal end portion 55 through the slit 58.

### [Seventh modification example]

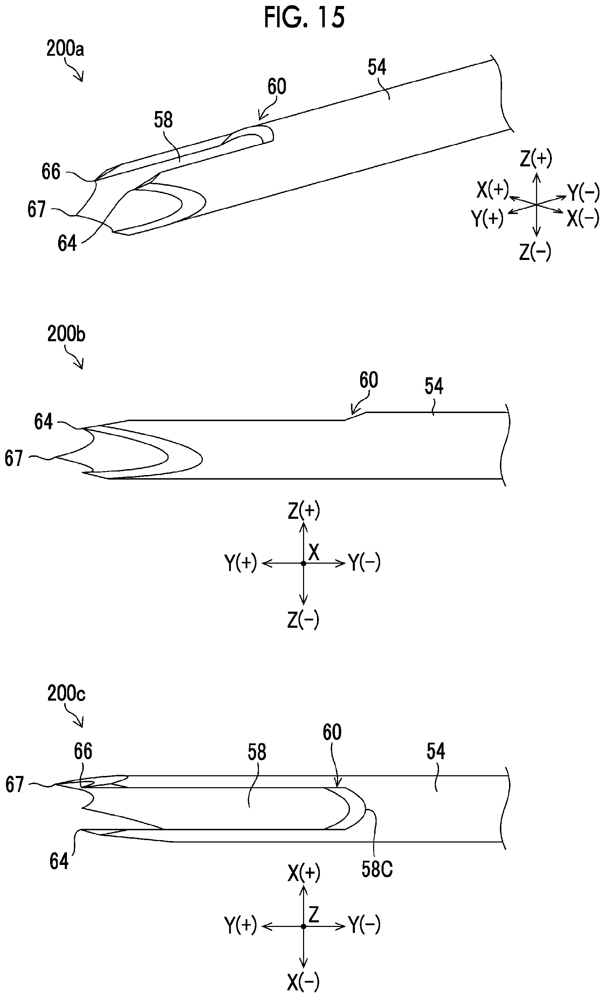

A seventh modification example shown in Fig. 15 is another modification example not falling under the scope of protection of the claims, in which a blade distal end point 67 is provided and is disposed not to face the slit 58 in the radial direction of the tube 54. 200a of Fig. 15 is a perspective view of the tube 54, 200b of Fig. 15 is a side view of the tube 54 in a case where the tube 54 is viewed from an X(-) side, and 200c of Fig. 15 is a top view of the tube 54 in a case where the tube 54 is viewed from a Z(+) side.

According to the seventh modification example, the blade distal end point 67 is formed closer to the distal end side of the tube 54 than the slit 58 in the axial direction of the tube 54 (Y direction). Further, the blade distal end point 67 is formed closer to the blade distal end point 66 than the blade distal end point 64 in the X direction and is formed at a portion of the tube 54 extending toward the distal end side from the blade distal end point 66 in the axial direction of the tube 54 (Y direction). Furthermore, the blade distal end point 67 is formed closer to the distal end side of the tube 54 than the blade distal end points 66 and 68.

According to the seventh modification example, the blade distal end point 67 is inserted into the biological tissue collection point T prior to the blade distal end points 64 and 66. However, since the tissue excision cutter 60 is provided at the proximal end 58C of the slit 58, it is possible to collect the amount of biological tissue enough to make a pathological confirmation diagnosis with a simple structure as in the case of the tube 54 shown in Fig. 5.

### [Eighth modification example]

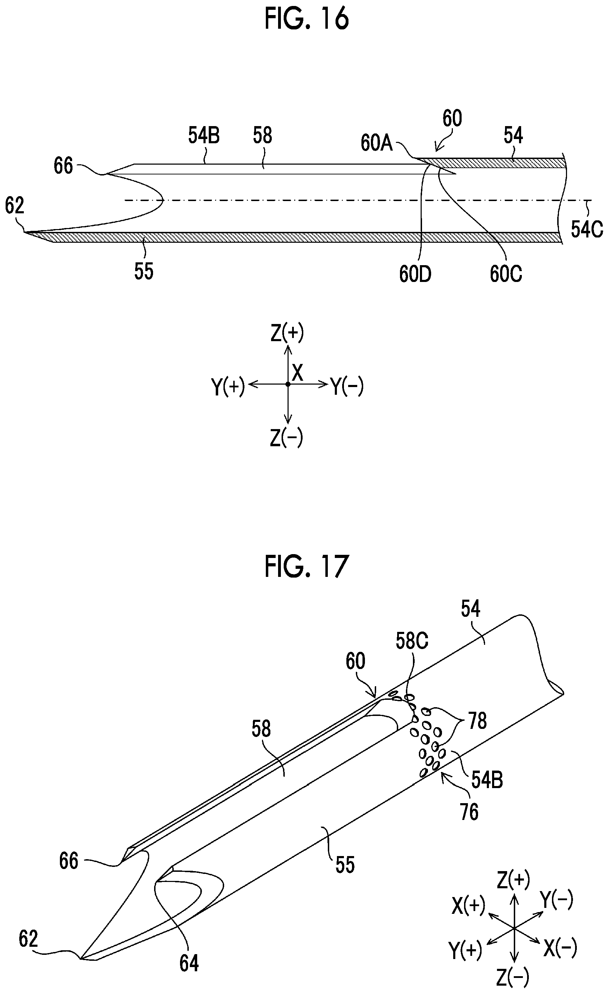

In an eighth modification example shown in Fig. 16, the tissue excision cutter 60 includes an inclined blade surface 60D that is inclined to be away from the central axis 54C of the tube 54 toward the distal end of the slit 58 from the proximal end 58C of the slit 58. Further, a blade 60A is formed at a portion of the tube 54 that protrudes outward from the outer peripheral surface 54B of the tube 54.

According to the eighth modification example, as the tube 54 is operated in the Y(+) direction, biological tissue entering the distal end portion 55 through the slit 58 can be excised by the blade 60A of the tissue excision cutter 60 and be collected in the distal end portion 55. In this case, the inclined blade surface 60D functions as a guide surface for pushing the excised biological tissue into the distal end portion 55.

Next, some aspects of an echo marker including the outer peripheral surface 54B of the tube 54 will be described. The echo marker is a marker that can allow the position of the tube 54 to be visually displayed in an ultrasound image. It is possible to visually confirm the position of the distal end portion 55 of the tube 54 by confirming the echo marker in the ultrasound image. In the description of the following aspects, the same members as or members similar to those of the tube 54 shown in Figs. 3 to 7 will be denoted by the same reference numerals as those of the tube 54.

As shown in Figs. 17 to 20, an echo marker 76 provided on the outer peripheral surface 54B of the tube 54 is formed of a plurality of small-diameter recessed portions 78 that are densely formed on the outer peripheral surface 54B. Accordingly, unevenness is formed on the outer peripheral surface 54B and ultrasonic visibility is enhanced by the unevenness, so that the position of the distal end portion 55 of the tube 54 can be confirmed in the ultrasound image.

### [First aspect]

First, in a first aspect shown in Fig. 17, the echo marker 76 is provided only around a periphery including the proximal end 58C of the slit 58.

According to the first aspect, it is possible to easily confirm the position of the proximal end 58C of the slit 58 by confirming the position of the echo marker 76 in the ultrasound image. Accordingly, since it is possible to easily confirm whether or not the slit 58 is inserted into the biological tissue by the total length thereof, it is possible to realize a safe and reliable procedure.

### [Second aspect]

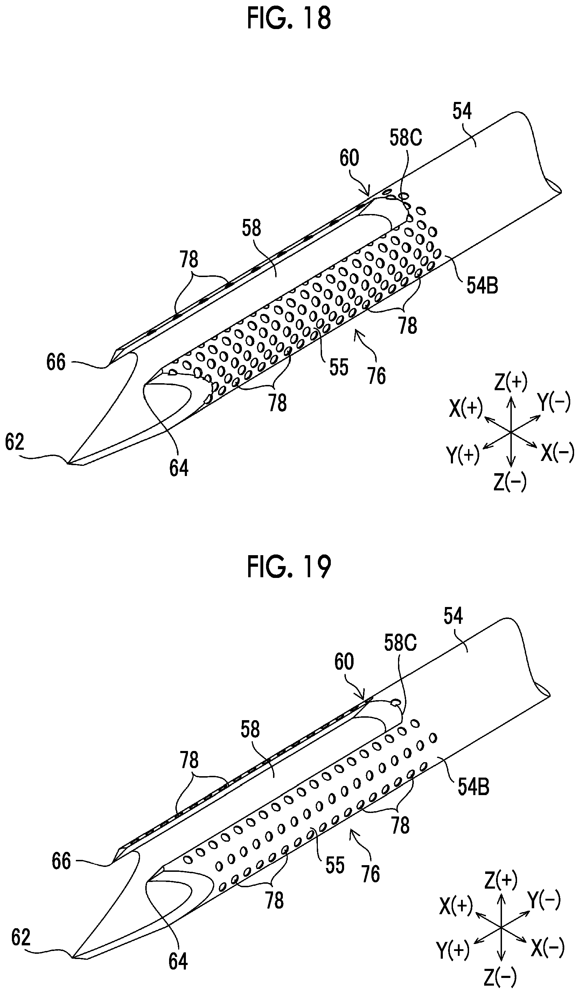

In a second aspect shown in Fig. 18, the echo marker 76 is provided in a region extending toward the distal end side of the tube 54 (Y(+) side) from a periphery, which includes the proximal end 58C of the slit 58, as a starting point. Specifically, the echo marker 76 is provided on the entire outer peripheral surface 54B of the tube 54 between the starting point and the blade distal end point 62. Although not shown in Fig. 18, the echo marker 76 is formed up to the surface of the blade distal end point 62 corresponding to a Z(-) side.

According to the second aspect, it is possible to easily confirm the position of the distal end portion 55, which includes the blade distal end point 62, by confirming the position of the distal end portion of the echo marker 76 in the ultrasound image. Further, it is possible to easily confirm the position of the proximal end 58C of the slit 58 by confirming the position of the proximal end portion of the echo marker 76. Accordingly, since it is possible to easily confirm whether or not the slit 58 is inserted into the biological tissue by the total length thereof, it is possible to realize a safe and reliable procedure.

### [Third aspect]

In a third aspect shown in Fig. 19, the echo marker 76 is provided in a region extending toward the distal end side of the tube 54 (Y(+) side) from a periphery, which includes the proximal end 58C of the slit 58, as a starting point. Specifically, the echo marker 76 is provided on the entire outer peripheral surface 54B of the tube 54 between the starting point and the blade distal end points 64 and 66.

According to the third aspect, it is possible to easily confirm the position of the distal end portion 55, which includes the blade distal end points 64 and 66, by confirming the position of the distal end portion of the echo marker 76 in the ultrasound image and to confirm the position of the proximal end 58C of the slit 58 by confirming the position of the proximal end portion of the echo marker 76. Accordingly, the same effects as those of the second aspect can be obtained.

### [Fourth aspect]

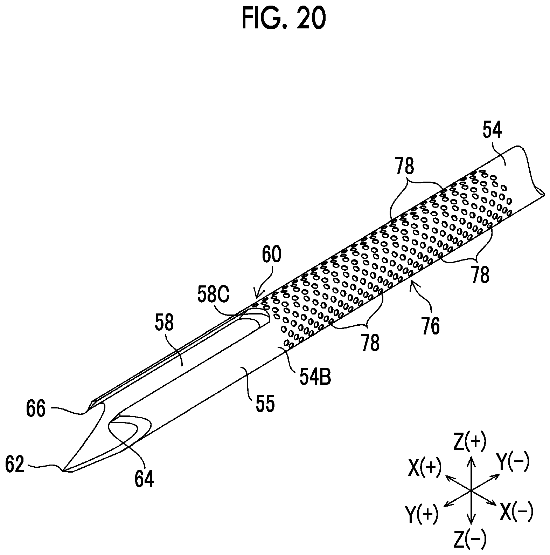

In a fourth aspect shown in Fig. 20, the echo marker 76 is provided in a region extending toward the proximal end side of the tube 54 (Y(-) side) from a periphery, which includes the proximal end 58C of the slit 58, as a starting point.

According to the fourth aspect, since it is possible to confirm the position of the proximal end 58C of the slit 58 by confirming the position of the distal end portion of the echo marker 76, the same effects as those of the first aspect can be obtained.

Each of the echo markers 76 shown in Figs. 17 to 20 is adapted to allow the position of the proximal end 58C of the slit 58 to be confirmed in order to particularly confirm whether or not the slit 58 is inserted into the biological tissue by the total length thereof. Such an echo marker 76 may be provided on the tubes 54 of the plurality of modification examples shown in Figs. 9 to 15.

### Explanation of References

10: insertion unit
11: angle part
12: distal end portion body
12a: inclined portion
12b: opening portion
13: endoscope observation part
14: ultrasonic observation part
15: illumination mechanism
16: ultrasonic transducer unit
17: ultrasonic oscillator
18: treatment tool outlet portion
19: connection pipe
20: flexible tube
21: treatment tool-insertion channel
30: tissue collecting device
31: insertion unit
32: operation unit
33: syringe
34: sheath
34A: distal end opening
35: biopsy needle
36: stylet
39: luer lock-operation part
39A: luer lock portion
40: sheath position-operation part
41: tube position-operation part
41A: distal end surface
42: stopper ring
43: screw
44: screw
45: luer lock portion
50: distal end opening portion
52: proximal end opening portion
54: tube
54A: inner peripheral surface
54B: outer peripheral surface
54C: central axis
55: distal end portion
56: puncture portion
58: slit
58B: wall portion
58C: proximal end
58A: wall portion
60: tissue excision cutter
60A: blade
60B: inclined blade surface
60C: blade distal end point
60D: inclined blade surface
62: blade distal end point
64: blade distal end point
66: blade distal end point
67: blade distal end point
68: blade surface
68A: blade
70: blade surface
70A: blade
72: blade distal end point
74: blade distal end point
76: echo marker
78: recessed portion

## Claims

1. A biopsy needle (35) that is to be inserted into an endoscope forceps channel, the biopsy needle comprising:
a hollow tube (54) which includes a distal end opening portion (50) and a proximal end opening portion (52) and of which the distal end opening portion and the proximal end opening portion communicate with each other,
wherein the tube includes a puncture portion (56) that is provided at a distal end portion (55) of the tube and has a plurality of blade distal end points, a slit (58) that extends toward the proximal end opening portion (52) from the distal end opening portion (50), and a tissue excision cutter (60) that is provided at a proximal end of the slit (58C) and is formed in a shape tapered toward a distal end of the slit from the proximal end of the slit,
wherein the puncture portion includes a first blade distal end point (62), a second blade distal end point (64), and a third blade distal end point (66),
in a case where the tube is viewed in a direction orthogonal to an axial direction of the tube, the first blade distal end point (62) is formed closer to a distal end side of the tube than the second blade distal end point (64) and the third blade distal end point (66), and
in a case where the tube is viewed in the axial direction of the tube, the first blade distal end point (62) is disposed to face the slit (58) in a radial direction of the tube, and the second blade distal end point (64) and the third blade distal end point (66) are disposed to face each other with the slit interposed therebetween,
**characterized in that**
the slit is an elongated through-hole that is formed in the shape of a line extending in the axial direction of the tube (54).

2. The biopsy needle according to claim 1, wherein the elongated through-hole of the slit is formed between a pair of wall portions (58A, 58B) facing each other in the direction orthogonal to the axial direction of the tube (54).

3. The biopsy needle according to any one of claims 1 to 2,
wherein the tissue excision cutter (60) includes at least one inclined blade surface (60B) that is inclined to approach a central axis (54C) of the tube (54) toward the distal end of the slit from the proximal end of the slit (58C).

4. The biopsy needle according to claim 3,
wherein a sharp blade (60A) is formed on the inclined blade surface (60B) and the blade is formed at a position where the blade does not protrude to an outside of the tube from an outer peripheral surface (54B) of the tube.

5. The biopsy needle according to any one of claims 3 to 4,
wherein the inclined blade surface (60B) has a shape like a rounded chisel.

6. The biopsy needle according to any one of claims 1 to 5,
wherein the distal end portion of the tube includes a first blade surface (68) that connects an outer peripheral surface (54B) of the tube to the first and second blade distal end points (62, 64) and has a normal line including a component toward an outside in the radial direction of the tube and a component toward the distal end side of the tube, and a second blade surface (70) that connects the outer peripheral surface (54B) of the tube to the first and third blade distal end points (62, 66) and has a normal line including a component toward the outside in the radial direction of the tube and a component toward the distal end side of the tube.

7. The biopsy needle according to any one of claims 1 to 6,
wherein the plurality of blade distal end points are formed on an inner peripheral surface (54A) of the tube.

8. The biopsy needle according to any one of claims 1 to 7,
wherein a width (B) of the slit in a circumferential direction of the tube is smaller than an inner diameter (ID) of the tube.

9. The biopsy needle according to any one of claims 1 to 8,
wherein an echo marker (76), which allows a position of the tube to be visually displayed in an ultrasound image, is provided on an outer peripheral surface (54B) of the tube.

10. The biopsy needle according to claim 9,
wherein the echo marker is provided only around a periphery including the proximal end (58C) of the slit.

11. The biopsy needle according to claim 9,
wherein the echo marker is provided in a region extending toward a distal end side of the tube from a periphery, which includes the proximal end of the slit, as a starting point.

12. The biopsy needle according to claim 9,
wherein the echo marker is provided in a region extending toward a proximal end side of the tube from a periphery, which includes the proximal end of the slit, as a starting point.

13. The biopsy needle according to any one of claims 9 to 12,
wherein the echo marker is formed in an uneven shape.

14. A tissue collecting device comprising:
the biopsy needle according to any one of claims 1 to 13; and
a suction member (33) that communicates with the proximal end opening portion of the biopsy needle and generates negative pressure in the biopsy needle.

## Patentansprüche

1. Biopsienadel (35), die in einen Endoskop-Zangenkanal einzuführen ist, wobei die Biopsienadel umfasst:
ein hohles Rohr (54), das einen distalen Endöffnungsabschnitt (50) und einen proximalen Endöffnungsabschnitt (52) enthält und von dem der distale Endöffnungsabschnitt und der proximale Endöffnungsabschnitt miteinander kommunizieren,
wobei das Rohr einen Punktionsabschnitt (56), der an einem distalen Endabschnitt (55) des Rohrs vorgesehen ist und mehrere distale Schneidenendpunkte aufweist, einen Schlitz (58), der sich von dem distalen Endöffnungsabschnitt (50) zu dem proximalen Endöffnungsabschnitt (52) hin erstreckt, und einen Gewebeexzisionsschneider (60), der an einem proximalen Ende des Schlitzes (58C) vorgesehen ist und in einer Form, die sich von dem proximalen Ende des Schlitzes zu einem distalen Ende des Schlitzes hin verjüngt, gebildet ist, enthält,
wobei der Punktionsabschnitt einen ersten distalen Schneidenendpunkt (62), einen zweiten distalen Schneidenendpunkt (64) und einen dritten distalen Schneidenendpunkt (66) enthält,
in einem Fall, in dem das Rohr in einer Richtung senkrecht zu einer Axialrichtung des Rohrs betrachtet wird, der erste distale Schneidenendpunkt (62) näher an einer distalen Endseite des Rohrs als der zweite distale Schneidenendpunkt (64) und der dritte distale Schneidenendpunkt (66) gebildet ist, und
in einem Fall, in dem das Rohr in der Axialrichtung des Rohrs betrachtet wird, der erste distale Schneidenendpunkt (62) so angeordnet ist, dass er dem Schlitz (58) in einer Radialrichtung des Rohrs zugewandt ist, und der zweite distale Schneidenendpunkt (64) und der dritte distale Schneidenendpunkt (66) so angeordnet sind, dass sie einander zugewandt sind, wobei dazwischen der Schlitz eingefügt ist,
**gekennzeichnet dadurch, dass**
der Schlitz ein länglicher Durchgangsloch ist, das in der Form einer Linie, die sich in der Axialrichtung des Rohrs (54) erstreckt, gebildet ist.

2. Biopsienadel nach Anspruch 1, wobei das längliche Durchgangsloch des Schlitzes zwischen einem Paar Wandabschnitten (58A, 58B) gebildet ist, die in der Richtung senkrecht zu der Axialrichtung des Rohrs (54) einander zugewandt sind.

3. Biopsienadel nach einem der Ansprüche 1 bis 2,
wobei der Gewebeexzisionsschneider (60) mindestens eine geneigte Schneidenfläche (60B) enthält, die so geneigt ist, dass sie sich von dem proximalen Ende des Schlitzes (58C) zu dem distalen Ende des Schlitzes hin einer Mittelachse (54C) des Rohrs (54) annähert.

4. Biopsienadel nach Anspruch 3,
wobei eine scharfe Schneide (60A) auf der geneigten Schneidenfläche (60B) gebildet ist und die Schneide an einer Position gebildet ist, an der die Schneide von einer Außenumfangsfläche (54B) des Rohrs zu einer Außenseite des Rohrs nicht vorsteht.

5. Biopsienadel nach einem der Ansprüche 3 bis 4,
wobei die geneigte Schneidenfläche (60B) eine Form wie ein abgerundeter Meißel aufweist.

6. Biopsienadel nach einem der Ansprüche 1 bis 5,
wobei der distale Endabschnitt des Rohrs eine erste Schneidenfläche (68), die eine Außenumfangsfläche (54B) des Rohrs mit den ersten und zweiten distalen Schneidenendpunkten (62, 64) verbindet und eine Normale, die eine Komponente zu einer Außenseite in der Radialrichtung des Rohrs hin und eine Komponente zu der distalen Endseite des Rohrs hin enthält, aufweist, und eine zweite Schneidenfläche (70), die die Außenumfangsfläche (54B) des Rohrs mit den ersten und dritten distalen Schneidenendpunkten (62, 66) verbindet und eine Normale, die eine Komponente zu der Außenseite in der Radialrichtung des Rohrs hin und eine Komponente zu der distalen Endseite des Rohrs hin enthält, aufweist, enthält.

7. Biopsienadel nach einem der Ansprüche 1 bis 6,
wobei die mehreren distalen Schneidenendpunkte an einer Innenumfangsfläche (54A) des Rohrs gebildet sind.

8. Biopsienadel nach einem der Ansprüche 1 bis 7,
wobei eine Breite (B) des Schlitzes in einer Umfangsrichtung des Rohrs kleiner als ein Innendurchmesser (ID) des Rohrs ist.

9. Biopsienadel nach einem der Ansprüche 1 bis 8,
wobei an einer Außenumfangsfläche (54B) des Rohrs eine Echomarkierung (76) vorgesehen ist, die es ermöglicht, dass eine Position des Rohrs in einem Ultraschallbild visuell angezeigt wird.

10. Biopsienadel nach Anspruch 9,
wobei die Echomarkierung nur um einen Umfang, der das proximale Ende (58C) des Schlitzes umfasst, vorgesehen ist.

11. Biopsienadel nach Anspruch 9,
wobei die Echomarkierung in einem Bereich vorgesehen ist, der sich von einem Umfang, der das proximale Ende des Schlitzes umfasst, als einem Startpunkt zu einer distalen Endseite des Rohrs hin erstreckt.

12. Biopsienadel nach Anspruch 9,
wobei die Echomarkierung in einem Bereich vorgesehen ist, der sich von einem Umfang, der das proximale Ende des Schlitzes umfasst, als einem Startpunkt zu einer proximalen Endseite des Rohrs hin erstreckt.

13. Biopsienadel nach einem der Ansprüche 9 bis 12,
wobei die Echomarkierung in einer ungleichmäßigen Form gebildet ist.

14. Gewebesammelvorrichtung, umfassend:
die Biopsienadel nach einem der Ansprüche 1 bis 13; und
ein Absaugelement (33), das mit dem proximalen Endöffnungsabschnitt der Biopsienadel kommuniziert und bei der Biopsienadel Unterdruck erzeugt.

## Revendications

1. Aiguille de biopsie (35) qui doit être insérée dans un canal de forceps d'endoscope, l'aiguille de biopsie comprenant :
un tube creux (54) qui inclut une partie d'ouverture d'extrémité distale (50) et une partie d'ouverture d'extrémité proximale (52) et dont la partie d'ouverture d'extrémité distale et la partie d'ouverture d'extrémité proximale communiquent entre elles,
dans laquelle le tube inclut une partie de perforation (56) qui est prévue à une partie d'extrémité distale (55) du tube et qui comporte une pluralité de points d'extrémité distale de lame, une fente (58) qui s'étend vers la partie d'ouverture d'extrémité proximale (52) à partir de la partie d'ouverture d'extrémité distale (50), et un cutter d'excision de tissu (60) qui est prévu à une extrémité proximale de la fente (58C) et qui est formé dans une forme effilée vers une extrémité distale de la fente à partir de l'extrémité proximale de la fente,
dans laquelle la partie de perforation inclut un premier point d'extrémité distal de lame (62), un deuxième point d'extrémité distal de lame (64) et un troisième point d'extrémité distal de lame (66),
dans un cas où le tube est vu dans une direction orthogonale à une direction axiale du tube, le premier point d'extrémité distal de lame (62) est formé plus près d'un côté d'extrémité distal du tube que le deuxième point d'extrémité distal de lame (64) et le troisième point d'extrémité distal de lame (66), et
dans un cas où le tube est vu dans la direction axiale du tube, le premier point d'extrémité distal de lame (62) est disposé pour faire face à la fente (58) dans une direction radiale du tube, et le deuxième point d'extrémité distal de lame (64) et le troisième point d'extrémité distal de lame (66) sont disposés pour se faire face avec la fente interposée entre eux,
**caractérisé en ce que**
la fente est un trou traversant allongé qui est formé sous la forme d'une ligne s'étendant dans la direction axiale du tube (54).

2. Aiguille de biopsie selon la revendication 1, dans laquelle le trou traversant allongé de la fente est formé entre une paire de parties de paroi (58A, 58B) se faisant face dans la direction orthogonale à la direction axiale du tube (54).

3. Aiguille de biopsie selon l'une quelconque des revendications 1 à 2,
dans laquelle le cutter d'excision de tissu (60) inclut au moins une surface de lame inclinée (60B) qui est inclinée pour se rapprocher d'un axe central (54C) du tube (54) vers l'extrémité distale de la fente depuis l'extrémité proximale de la fente (58C).

4. Aiguille de biopsie selon la revendication 3,
dans laquelle une lame tranchante (60A) est formée sur la surface inclinée de lame (60B) et la lame est formée à une position où la lame ne dépasse pas à un extérieur du tube depuis une surface périphérique externe (54B) du tube.

5. Aiguille de biopsie selon l'une quelconque des revendications 3 à 4,
dans laquelle la surface de lame inclinée (60B) a une forme semblable à un ciseau arrondi.

6. Aiguille de biopsie selon l'une quelconque des revendications 1 à 5,
dans laquelle la partie d'extrémité distale du tube inclut une première surface de lame (68) qui relie une surface périphérique externe (54B) du tube aux premier et deuxième points d'extrémité distale de lame (62, 64) et comporte une ligne normale incluant une composante vers un extérieur dans la direction radiale du tube et une composante vers le côté d'extrémité distale du tube, et une deuxième surface de lame (70) qui relie la surface périphérique externe (54B) du tube aux premier et troisième points d'extrémité distale de lame (62, 66) et comporte une ligne normale incluant une composante vers l'extérieur dans la direction radiale du tube et une composante vers le côté d'extrémité distale du tube.

7. Aiguille de biopsie selon l'une quelconque des revendications 1 à 6,
dans laquelle la pluralité de points d'extrémité distale de lame sont formés sur une surface périphérique interne (54A) du tube.

8. Aiguille de biopsie selon l'une quelconque des revendications 1 à 7,
dans laquelle une largeur (B) de la fente dans une direction circonférentielle du tube est inférieure à un diamètre interne (ID) du tube.

9. Aiguille de biopsie selon l'une quelconque des revendications 1 à 8,
dans laquelle un marqueur d'écho (76), qui permet d'afficher visuellement une position du tube dans une image ultrasonore, est prévu sur une surface périphérique externe (54B) du tube.

10. Aiguille de biopsie selon la revendication 9,
dans laquelle le marqueur d'écho est prévu uniquement autour d'une périphérie incluant l'extrémité proximale (58C) de la fente.

11. Aiguille de biopsie selon la revendication 9,
dans laquelle le marqueur d'écho est prévu dans une région s'étendant vers un côté distal du tube à partir d'une périphérie, qui inclut l'extrémité proximale de la fente, comme point de départ.

12. Aiguille de biopsie selon la revendication 9,
dans laquelle le marqueur d'écho est prévu dans une région s'étendant vers un côté d'extrémité proximale du tube à partir d'une périphérie, qui inclut l'extrémité proximale de la fente, comme point de départ.

13. Aiguille de biopsie selon l'une quelconque des revendications 9 à 12,
dans laquelle le marqueur d'écho est formé dans une forme irrégulière.

14. Dispositif de collecte de tissus comprenant :
l'aiguille de biopsie selon l'une quelconque des revendications 1 à 13 ; et
un élément d'aspiration (33) qui communique avec la partie d'ouverture d'extrémité proximale de l'aiguille de biopsie et génère une pression négative dans l'aiguille de biopsie.
